# EUROPEAN PATENT SPECIFICATION

(11) **EP 1 595 509 B1**
(45) Date of publication and mention of the grant of the patent: **29.10.2008**
(21) Application number: 05017281.6
(22) Date of filing: 06.04.2001
(51) Int. Cl.: A61B 18/14

(54) **Vessel sealing instrument**
Gefässversiegelnde Vorrichtung
Instrument de suture de vaisseaux

(43) Date of publication of application: 16.11.2005
(62) Divisional of application: 01923224.8
(73) Proprietor: Covidien AG, 8212 Neuhausen am Rheinfall (CH)
(72) Inventor: Tetzlaff, Philip Mark, Superior, CO 80027 (US); Mihaichuk, Carolyn Heather, Erie, CO 80516 (US); Kerr, Duane Eugene, Bethoud, CO 80513 (US); Schechter, Dave A., Longmont, CO 80503 (US); Sherman, Jon, Cincinnati, OH 45240 (US); Richardson, Ted, Cincinnati, OH 45255 (US); Drach, Greg, Liberty Township, OH 45011 (US)
(74) Representative: HOFFMANN EITLE

(56) References cited:
- WO-A-00/24330
- WO-A-99/12488
- US-A- 5 499 997
- US-A- 5 573 534
- US-A- 5 658 281
- US-A- 5 954 720

## Description

### BACKGROUND

The present disclosure relates to forceps used for open surgical procedures. More particularly, the present disclosure relates to a forceps which applies a combination of mechanical clamping pressure and electrosurgical current to seal tissue.

### Technical Field

A hemostat or forceps is a simple plier-like tool which uses mechanical action between its jaws to constrict vessels and is commonly used in open surgical procedures to grasp, dissect and/or clamp tissue. Electrosurgical forceps utilize both mechanical clamping action and electrical energy to effect hemostasis by heating the tissue and blood vessels to coagulate, cauterize and/or seal tissue.

Certain surgical procedures require sealing and cutting blood vessels or vascular tissue. Several journal articles have disclosed methods for sealing small blood vessels using electrosurgery. An article entitled Studies on Coagulation and the Development of an Automatic Computerized Bipolar Coagulator, J. Neurosurg., Volume 75, July 1991, describes a bipolar coagulator which is used to seal small blood vessels. The article states that it is not possible to safely coagulate arteries with a diameter larger than 2 to 2.5 mm. A second article is entitled Automatically Controlled Bipolar Electrocoagulation - "COA-COMP", Neurosurg. Rev. (1984), pp. 187-190, describes a method for terminating electrosurgical power to the vessel so that charring of the vessel walls can be avoided.

By utilizing an electrosurgical forceps, a surgeon can either cauterize, coagulate/desiccate, reduce or slow bleeding and/or seal vessels by controlling the intensity, frequency and duration of the electrosurgical energy applied to the tissue. Generally, the electrical configuration of electrosurgical forceps can be categorized in two classifications: 1) monopolar electrosurgical forceps; and 2) bipolar electrosurgical forceps.

Monopolar forceps utilize one active electrode associated with the clamping end effector and a remote patient return electrode or pad which is typically attached externally to the patient. When the electrosurgical energy is applied, the energy travels from the active electrode, to the surgical site, through the patient and to the return electrode.

Bipolar electrosurgical forceps utilize two generally opposing electrodes which are disposed on the inner opposing surfaces of the end effectors and which are both electrically coupled to an electrosurgical generator. Each electrode is charged to a different electric potential. Since tissue is a conductor of electrical energy, when the effectors are utilized to grasp tissue therebetween, the electrical energy can be selectively transferred through the tissue.

In order to effect a proper seal with larger vessels, two predominant mechanical parameters must be accurately controlled - the pressure applied to the vessel and the gap between the electrodes both of which affect thickness of the sealed vessel. More particularly, accurate application of the pressure is important to oppose the walls of the vessel, to reduce the tissue impedance to a low enough value that allows enough electrosurgical energy through the tissue, to overcome the forces of expansion during tissue heating and to contribute to the end tissue thickness which is an indication of a good seal. It has been determined that a fused vessel wall is optimum between 0.0254 and 0.127 mm (0.001 and 0.005 inches). Below this range, the seal may shred or tear and above this range the lumens may not be properly or effectively sealed.

With respect to smaller vessel, the pressure applied to the tissue tends to become less relevant whereas the gap distance between the electrically conductive surfaces becomes more significant for effective sealing. In other words, the chances of the two electrically conductive surfaces touching during activation increases as the vessels become smaller.

Electrosurgical methods may be able to seal larger vessels using an appropriate electrosurgical power curve, coupled with an instrument capable of applying a large closure force to the vessel walls. It is thought that the process of coagulating small vessels is fundamentally different than electrosurgical vessel sealing. For the purposes herein, "coagulation" is defined as a process of desiccating tissue wherein the tissue cells are ruptured and dried and vessel sealing is defined as the process of liquefying the collagen in the tissue so that it reforms into a fused mass. Thus, coagulation of small vessels is sufficient to permanently close them. Larger vessels need to be sealed to assure permanent closure.

Numerous bipolar electrosurgical forceps have been proposed in the past for various open surgical procedures. However, some of these designs may not provide uniformly reproducible pressure to the blood vessel and may result in an ineffective or non-uniform seal. For example, U.S. Patent No. 2,176,479 to Willis, U.S. Patent Nos. 4,005,714 and 4,031,898 to Hiltebrandt, U.S. Patent Nos. 5,827,274, 5,290,287 and 5,312,433 to Boebel et al., U.S. Patent Nos. 4,370,980, 4,552,143, 5,026,370 and 5,116,332 to Lottick, U.S. Patent No. 5,443,463 to Stem et al., U.S. Patent No. 5,484,436 to Eggers et al. and U.S. Patent No. 5,951,549 to Richardson et al., all relate to electrosurgical instruments for coagulating, cutting and/or sealing vessels or tissue.

Many of these instruments include blade members or shearing members which simply cut tissue in a mechanical and/or electromechanical manner and are relatively ineffective for vessel sealing purposes. Other instruments rely on clamping pressure alone to procure proper sealing thickness and are not designed to take into account gap tolerances and/or parallelism and flatness requirements which are parameters which, if properly controlled, can assure a consistent and effective tissue seal. For example, it is known that it is difficult to adequately control thickness of the resulting sealed tissue by controlling clamping pressure alone for either of two reasons: 1) if too much force is applied, there is a possibility that the two poles will touch and energy will not be transferred through the tissue resulting in an ineffective seal; or 2) if too low a force is applied, a thicker less reliable seal is created.

As mentioned above, in order to properly and effectively seal larger vessels, a greater closure force between opposing jaw members is required. It is known that a large closure force between the jaws typically requires a large moment about the pivot for each jaw. This presents a challenge because the jaw members are typically affixed with pins which are positioned to have a small moment arms with respect to the pivot of each jaw member. A large force, coupled with a small moment arm, is undesirable because the large forces may shear the pins. As a result, designers must compensate for these large closure forces by either designing instruments with metal pins and/or by designing instruments which at least partially offload these closure forces to reduce the chances of mechanical failure. As can be appreciated, if metal pivot pins are employed, the metal pins must be insulated to avoid the pin acting as an alternate current path between the jaw members which may prove detrimental to effective sealing.

Increasing the closure forces between electrodes may have other undesirable effects, e.g., it may cause the opposing electrodes to come into close contact with one another which may result in a short circuit and a small closure force may cause pre-mature movement of the issue during compression and prior to activation.

Thus, a need exists to develop a bipolar forceps which effectively seals vascular tissue and solves the aforementioned problems by providing an instrument which enables a large closure force between the opposing jaws members, reduces the chances of short circuiting the opposing jaws during activation and assists in manipulating, gripping and holding the tissue prior to and during activation.

US Patent 5,954,720 discloses a bipolar electrosurgical instrument provided with one metal and one ceramic end effector.

### SUMMARY

The invention is defined by the independent claim, the preamble of which is based on WO 00/24330. The dependent claims are directed towards preferred embodiments.

The present disclosure relates to a bipolar electrosurgical instrument for use in open surgery which includes first and second shafts one of which is connectable to a source of electrosurgical energy. Each shaft includes a jaw member extending from a distal end thereof and a handle disposed at a proximal end thereof for effecting movement of the jaw members relative to one another from a first, open position wherein the jaw members are disposed in spaced relation relative to one another to a second, closed position wherein the jaw members cooperate to grasp tissue therebetween. The source of electrical energy effects first and second electrical potentials in the respective jaw members such that the jaw members are capable of selectively conducting energy through tissue held therebetween to effect a seal.

Preferably, the first and second electrical potentials are created at the jaw members through the first shaft. For example, in one embodiment, the first electrical potential is transmitted through the first shaft by a lead having a terminal end which electrically interfaces with a distal connector which connects a first jaw member to the first electrical potential. The second electrical potential is transmitted through the first shaft by a tube disposed within the first shaft which connects the second jaw member to the second electrical potential.

The first and second jaw members are connected about a pivot pin. The distal connector is preferably interposed between the jaw members and includes a series of flanges which are dimensioned to prevent the emanation of stray currents from the electrically conductive sealing surfaces of the jaw members during activation.

The distal connector includes a spring washer or wave washer which acts as an electrical intermediary between the terminal end and the jaw member. In one embodiment, the spring washer is beveled to enhance the electrical interface between the terminal end and the jaw member, i.e., beveling causes the spring washer to rotate relative the terminal end during movement of the jaw members from the first to second positions which provides a self-cleaning, enhanced running electrical contact between the terminal end and the jaw member.

Preferably, the distal connector is made from an insulative substrate and is disposed between the jaw members for electrically isolating the first and second potentials. In one embodiment, the distal connector includes a first surface having at least one recess defined therein which is dimensioned to receive at least a portion of the terminal end of the lead.

In yet another embodiment, one of the jaw members includes a skirt which is dimensioned to prevent exposure of the terminal end during all angles of operation, i.e., when the jaw members are disposed in the first position, the second position and/or during operative movement therebetween.

The lead preferably includes a inner core made from a solid or multi-strand electrically conductive material, e.g., copper/aluminum wire, which is surrounded by an insulative, non-conductive coating, e.g., plastic. In one embodiment, the terminal or distal end of the electrically conductive material is flattened, i.e., "flat-formed", and is dimensioned to substantially encircle a boss which extends from the surface of the distal connector. Preferably, the boss is designed to electrically insulate the terminal end of the lead from the pivot pin.

In another embodiment, at least one non-conductive stop member is disposed on an electrically conductive sealing surface of one of the jaw members. The stop members are designed to control/regulate the distance, i.e., gap, between the jaw members when tissue is held therebetween during activation

### BRIEF DESCRIPTION OF THE DRAWINGS

Various embodiments of the subject instrument are described herein with reference to the drawings wherein:
Fig. 1 is a left, perspective view of a forceps according to the present disclosure;
Fig. 2 is an enlarged, perspective view of an end effector assembly of the forceps of Fig. 1 shown in open configuration;
Fig. 3 is an enlarged, perspective view of the end effector assembly of the forceps of Fig. 1 shown in closed configuration;
Fig. 4A is an exploded view of the forceps according to the present disclosure;
Fig. 4B is an enlarged, exploded view of the end effector assembly of Fig. 4A showing the electrical connection of a distal electrical connector for supplying electrical energy to the end effector assembly;
Fig. 5 is an enlarged, top perspective view of a lower jaw member of forceps with the distal connector seated thereon;
Fig. 6 is a right, perspective view of the forceps of Fig. 1 shown grasping a tissue structure;
Fig. 7 is a enlarged view of the indicated area of detail in Fig. 4A showing a proximal electrical interface/connector for supplying electrical energy to the end effector assembly; and
Fig. 8 is a cross section of the forceps of Fig. 6 showing the electrical feed path of a first lead having a first electrical potential and showing the electrical connection of the proximal electrical interface of Fig. 7 with a second lead having a second electrical potential.

### DETAILED DESCRIPTION

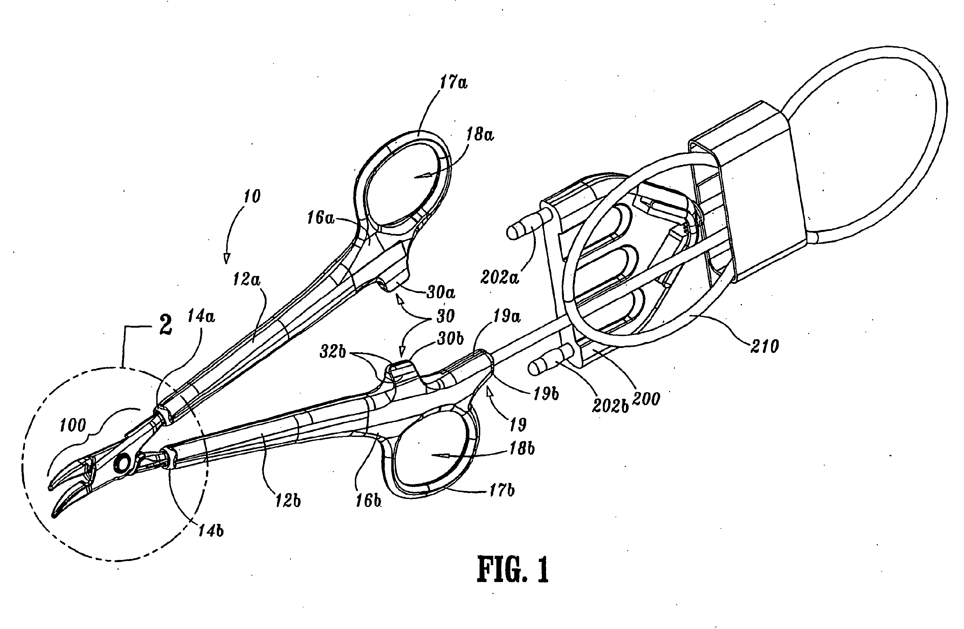

Referring now to Figs. 1-4, a forceps 10 for use with open surgical procedures includes elongated shaft portions 12a and 12b each having a proximal end 16a and 16b, respectively, and a distal end 14a and 14b, respectively. In the drawings and in the descriptions which follow, the term "proximal", as is traditional, will refer to the end of the forceps 10 which is closer to the user, while the term "distal" will refer to the end which is further from the user.

The forceps 10 includes an end effector assembly 100 which attaches to distal ends 14a and 14b of shafts 12a and 12b, respectively. As explained in more detail below, the end effector assembly 100 includes pair of opposing jaw members 110 and 120 which are pivotably connected about a pivot pin 150.

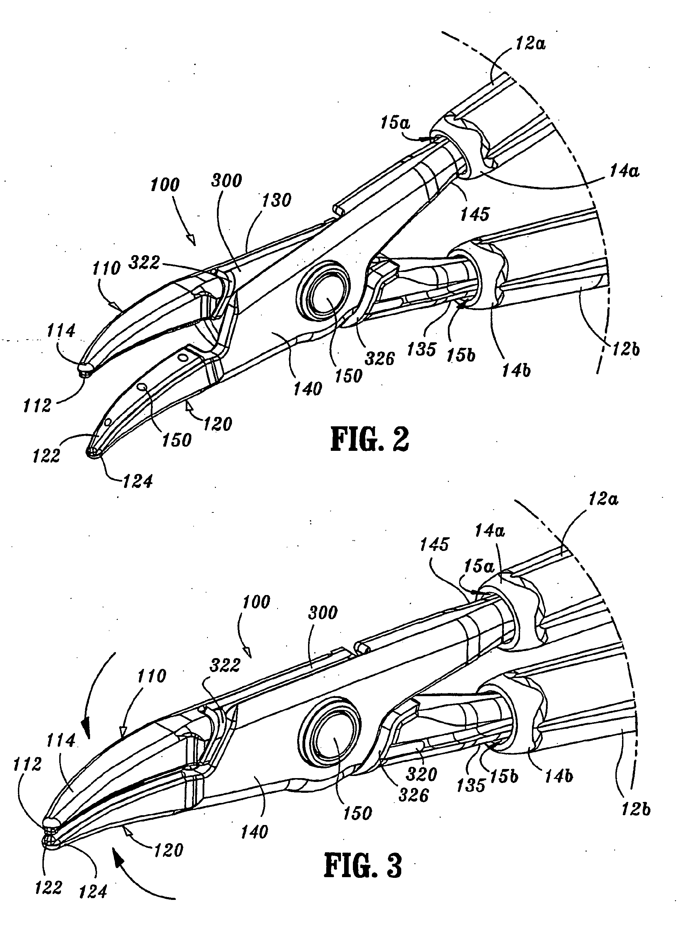

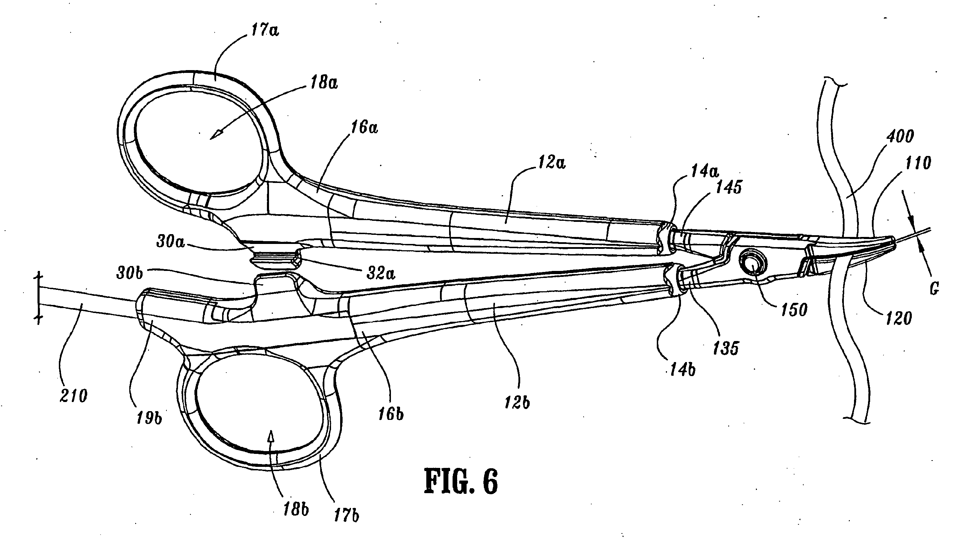

Preferably, each shaft 12a and 12b includes a handle 17a and 17b disposed at the proximal end 16a and 16b thereof which each define a finger hole 18a and 18b, respectively, therethrough for receiving a finger of the user. As can be appreciated, finger holes 18a and 18b facilitate movement of the shafts 12a and 12b relative to one another which, in turn, pivot the jaw members 110 and 120 from an open position (Fig. 2) wherein the jaw members 110 and 120 are disposed in spaced relation relative to one another to a clamping or closed position (Fig. 3) wherein the jaw members 110 and 120 cooperate to grasp tissue 400 (Fig. 6) therebetween.

A ratchet 30 is preferably included for selectively locking the jaw members 110 and 120 relative to one another at various positions during pivoting. As best shown in Fig. 6, a first ratchet interface, e.g., 30a, extends from the proximal end 16a of shaft member 12a towards a second ratchet interface 30b in a generally vertically aligned manner such that the inner facing surfaces of each ratchet 30a and 30b abut one another upon closure about the tissue 400. Preferably, each ratchet interface 30a and 30b includes a plurality of flanges 32a and 32b, respectively, which projects from the inner facing surface of each ratchet interface 30a and 30b such that the ratchet interfaces 30a and 30b interlock in at least one position. In the embodiment shown in Fig. 6, the ratchet interfaces 30a and 30b interlock at several different positions.

Preferably, each position associated with the cooperating ratchet interfaces 30a and 30b holds a specific, i.e., constant, strain energy in the shaft members 12a and 12b which, in turn, transmits a specific closing force to the jaw members 110 and 120. It is envisioned that the ratchet 30 may include graduations or other visual markings which enable the user to easily and quickly ascertain and control the amount of closure force desired between the jaw members. A design without a ratchet system or similar system would require the user to hold the jaw members 110 and 120 together by applying constant force to the handles 17a and 17b which may yield inconsistent results.

As best illustrated in Fig. 1, one of the shafts, e.g., 12b, includes a proximal shaft connector 19 which is designed to connect the forceps 10 to a source of electrosurgical energy such as an electrosurgical generator (not shown). More particularly, proximal shaft connector 19 is formed by a cover 19a and a flange 19b which extends proximally from shaft 12b. Preferably, cover 19a and flange 19b mechanically cooperate to secure an electrosurgical cable 210 to the forceps 10 such that the user may selectively apply electrosurgical energy as needed.

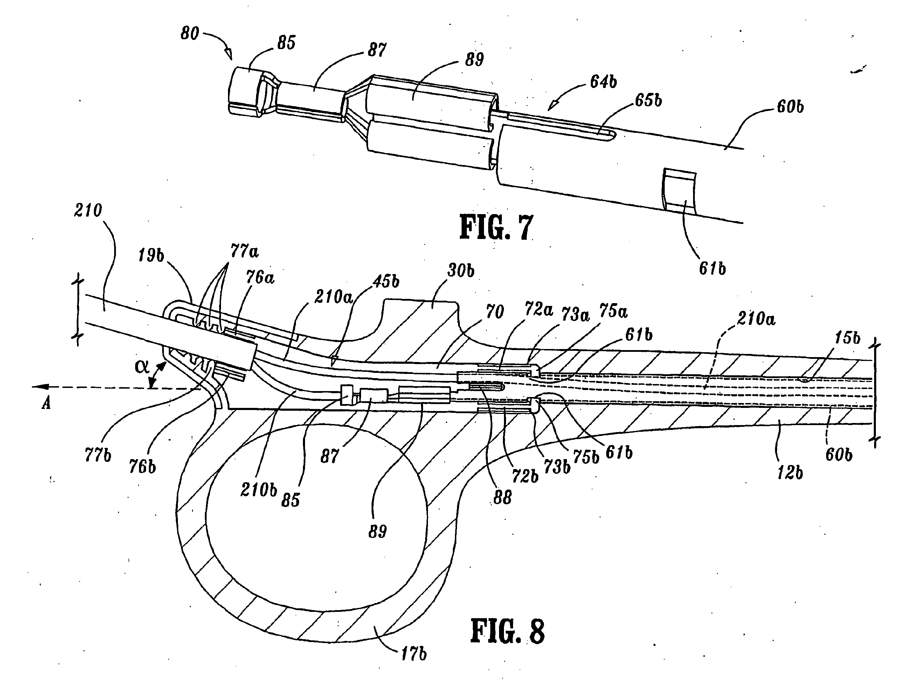

The proximal end of the cable 210 includes a plug 200 having a pair of prongs 202a and 202b which are dimensioned to electrically and mechanically engage the electrosurgical energy generator. As explained in more detail below with respect to Fig. 8, the distal end of the cable 210 is secured to the proximal shaft connector 19 of shaft 12b by a plurality of finger-like clamping members 77a and 77b and a cable crimp having opposing fingers 76a and 76b. The interior of cable 210 houses a pair of leads 210a and 210b which conduct the different electrical potentials from the electrosurgical generator to the jaw members 110 and 120 as explained in greater detail below.

As best seen in Figs. 2 - 4B, the two opposing jaw members 110 and 120 of the end effector assembly 100 are pivotable about pin 150 from the open position to the closed position for grasping tissue 400 therebetween. Jaw members 110 and 120 are generally symmetrical and include similar component features which cooperate to permit facile rotation about pivot pin 150 to effect the grasping and sealing of tissue 400. As a result and unless otherwise noted, jaw member 110 and the operative features associated therewith will initially be described herein in detail and the similar component features with respect to jaw member 120 will be briefly summarized thereafter.

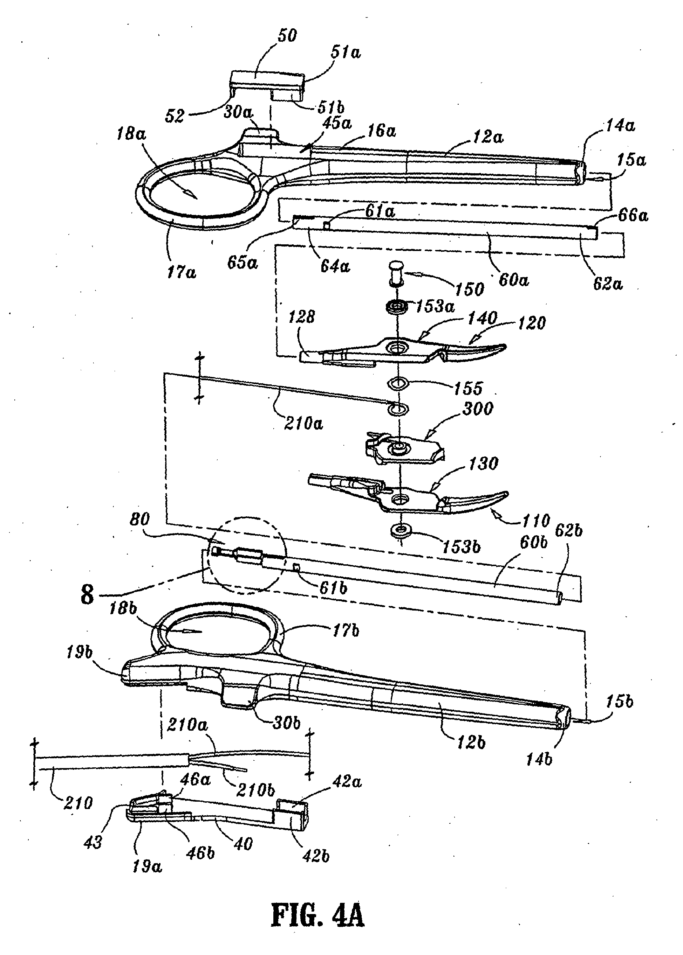

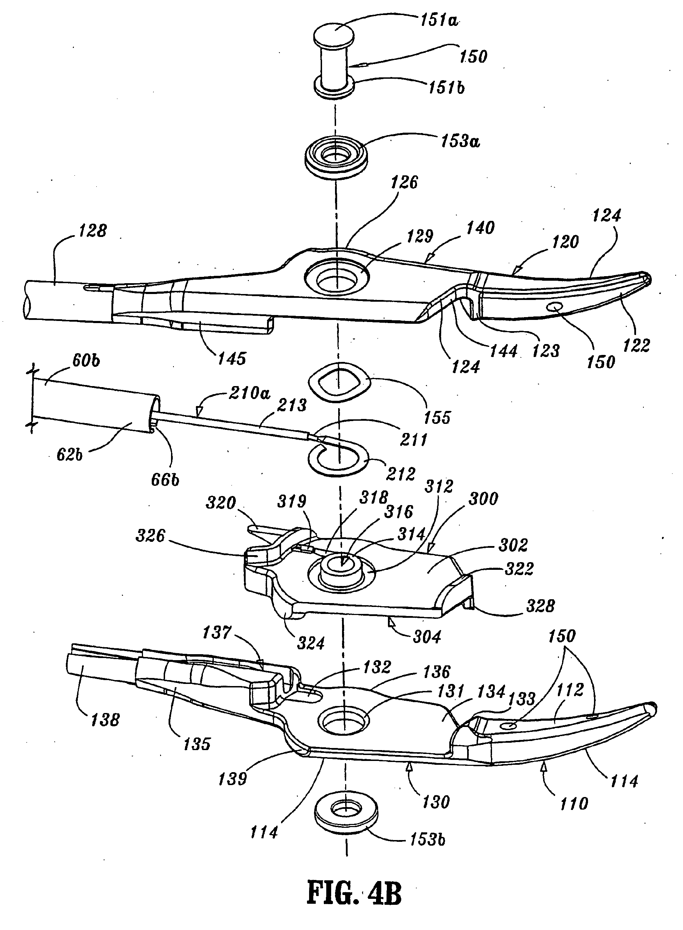

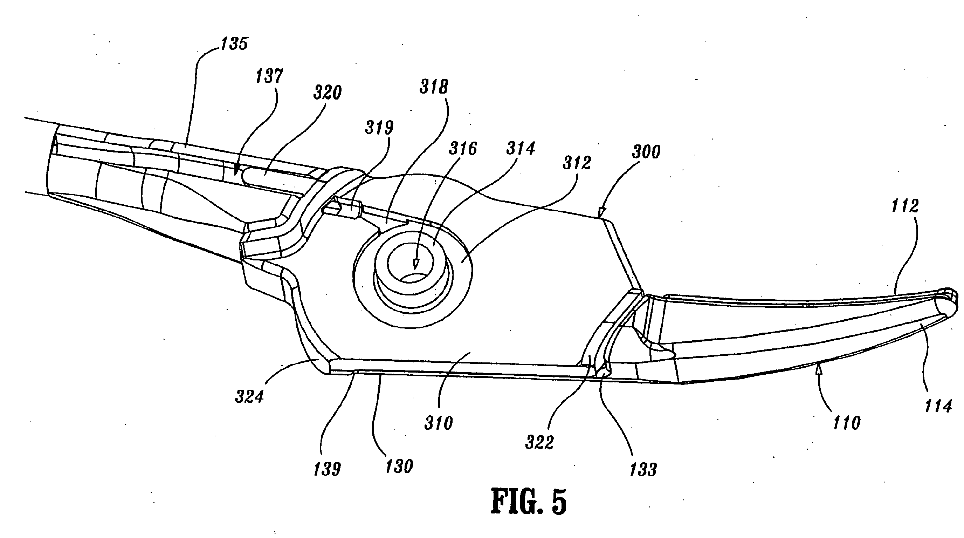

Jaw member 110 includes an insulated outer housing 114 which is dimensioned to mechanically engage an electrically conductive sealing surface 112 and a proximally extending flange 130 which is dimensioned to seat a distal connector 300 which is described in more detail below with respect to Figs. 4A, 4B and 5. Preferably, outer insulative housing 114 extends along the entire length of jaw member 110 to reduce alternate or stray current paths during sealing and/or incidental burning of tissue 400. The inner facing surface of flange 130 includes an electrically conductive plate 134 (Fig. 4B) which conducts electrosurgical energy to the electrically conductive sealing surface 112 upon activation.

Likewise, jaw member 120 include similar elements which include: an outer housing 124 which engages an electrically conductive sealing surface 122; a proximally extending flange 140 which seats the opposite face of the distal connector 300; an electrically conductive plate 144 which conducts electrosurgical energy to the electrically conductive sealing surface 122 upon activation.

It is envisioned that one of the jaw members, e.g., 110, includes at least one stop member 150 disposed on the inner facing surface of the electrically conductive sealing surface 112 (and/or 122). The stop member(s) is preferably designed to facilitate gripping and manipulation of tissue 400 and to define a gap "G" (Fig. 6) between opposing jaw members 110 and 120 during sealing. A detailed discussion of these and other envisioned stop members 150 as well as various manufacturing and assembling processes for attaching, disposing, depositing and/or affixing the stop members 150 to the electrically conductive sealing surfaces 112, 122 are described in commonly-assigned, co-pending U.S. Application Serial No. 10/338,953 entitled "BIPOLAR ELECTROSURGICAL FORCEPS WITH NON-CONDUCTIVE STOP MEMBERS" published as US 2003 181910.

Fig. 4A shows an exploded view of the various components of the forceps 10 and the inter-operative relationships among the same. More particularly and in addition to the components described above with respect to Figs. 1-3 above, shaft 12a is preferably hollow to define a longitudinal channel 15a disposed therethrough which is dimensioned to receive a tube 60a therein. Tube 60a includes a proximal end 64a, a distal end 62a and at least one mechanical interface 61 a disposed therebetween. Shaft 12a also includes a cover plate 50 which is designed for snap-fit engagement within an aperture/cavity 45a defined through the outer surface of shaft 12a. Cover plate 50 includes a series of opposing flanges 51 a and 51 b which extend therefrom which are dimensioned to secure the tube 60a within shaft 12a as described below. A second flange 52 secures the cover plate 50 to the shaft 12a.

During assembly, the proximal end 64a of tube 60a is slideable incorporated within channel 15a such that mechanical interface 61 a is poised for engagement with cover plate 50. Cover plate 50 is then snapped into cavity 45a such that flanges 51 a and 51 b secure tube 60a within shaft 12a. It is envisioned that the cavity 45a of shaft 12a may include at least one detent (not shown) which engages mechanical interface 61a disposed along the outer surface of tube 60a to limit / prevent rotation of the tube 60a relative to the shaft 12a. This cooperative relationship is shown by way of example with respect to detents 75a and 75b and interfaces (e.g., notches) 61b of shaft 12b in Fig. 8. In this instance, flanges 51 a and 51 b (much like flanges 42a and 42b of cover plate 40 in Fig. 8) hold the detents 75a and 75b in Fig. 8) in secure engagement within the notch(es) 61 a to prevent rotational and/or longitudinal movement of the tube 60a within the channel 15a.

Preferably, the proximal-most end of tube 60a includes a slit-like interface 65a which mechanically engages a corresponding tongue 88a extending from the inner surface of shaft 12a within cavity 45a. It is envisioned that tongue 88a also prevents rotational movement of the tube 60a within the shaft 12a. Alternatively, slit 65a may be formed to allow radial contraction and expansion of the tube 60a to promote friction-fit engagement between the tube 60a and the shaft 12a. Other interfaces are also envisioned which will facilitate engagement of the shaft 12a and the tube 60a, e.g., snap-fit, spring-lock, locking tabs, screw-like interface, tongue and groove, etc.

The distal end 62a of tube 60a is preferably dimensioned to engage jaw member 120, i.e., the distal end 62a includes a slit-like interface 66a which promotes simple, secure friction-fit engagement of the tube 60a with the jaw member 120. More particularly and as mentioned above, jaw member 120 includes a proximally extending flange 130 having a sleeve 128 extending proximally therefrom which is dimensioned such that, upon insertion of the sleeve 128 within distal end 62a, slit-like interface 66a expands radially outwardly and securely locks the jaw member 120 to tube 60a. Again, other methods of attachment are also envisioned which would serve the same purpose, e.g., snap-locks, locking tabs, spring-locks, screw-like interface, tongue and groove, etc.

As can be appreciated by the present disclosure, the arrangement of shaft 12b is slightly different from shaft 12a as shown best in Figs. 4B, 7 and 8. More particularly, shaft 12b is also hollow to define a channel 15b therethrough and is dimensioned to receive a tube 60b therein. Tube 60b includes a proximal end 64b and a distal end 62b which attach in a generally similar fashion as their counterpart components with respect to shaft 12a. For example, the proximal end 64b of tube 60b is slideable incorporated within channel 15b such that a mechanical interface 61 b disposed on the outer surface of tube 60b is poised for engagement with a cover plate 40 (Figs. 4A and 8).

Preferably and since the forceps 10 is uniquely designed to incorporate all of the electrical interfaces and connections within and along a single shaft, e.g., 12b, shaft 12b includes a slightly larger cavity 45b defined therein for housing and securing the various electrical connections associated with the forceps 10 as described below. For example, cover plate 40 is dimensioned slightly differently than cover plate 50 mostly due to the spatial considerations which must be taken into account for incorporation of the various internally disposed electrical connections. However, cover plate 40 does snap atop shaft 12b such that a pair of flanges 42a and 42b secure tube 60b within shaft 12b in a similar manner as described above. For example, Figure 8 shows a pair of detents 75a and 75b disposed within the cavity 45b of shaft 12b which engage a corresponding number of mechanical interfaces 61 b disposed along the outer surface of tube 60b to limit / prevent rotation of the tube 60b relative to the shaft 12b. When assembled, each flange 42a and 42b is pushed into a corresponding groove 73a and 73b, respectively, which effectively maintain / hold the detents 75a and 75b in secure engagement within the notches 61 b to prevent rotational and/or longitudinal movement of the tube 60b within the channel 15b.

End 64b of tube 60b also includes a slit-like interface 65b which mechanically engages a corresponding tongue 88b extending from the inner surface of shaft 12b within cavity 45b. It is envisioned that tongue 88a also prevents rotational movement of the tube 60b within the shaft 12b. Alternatively, slit 65b may be formed to allow radial contraction and expansion of the tube 60b to promote friction-fit engagement between the tube 60b and the shaft 12b.

Unlike tube 60a, tube 60b is designed as an electrical conduit for transmitting electrosurgical energy to jaw member 110 which is explained in more detail below with respect to Figs. 7 and 8. The distal end 62b of tube 60b is preferably dimensioned to engage jaw member 110, i.e., the distal end 62b includes a slit-like interface 66b which promotes simple, secure friction-fit engagement of the tube 60b with the jaw member 110. This is best illustrated in Fig. 4B which shows proximally extending flange 130 of jaw member 110 having a terminal sleeve 138 which extends therefrom. Terminal sleeve 138 is dimensioned such that, upon insertion of the terminal sleeve 138 within distal end 62b, slit-like interface 66b expands radially outwardly and securely locks the jaw member 110 to tube 60b.

As can be appreciated, terminal end 138 is at least partially made from an electrically conductive material such that an electrosurgical potential is effectively conducted from the tube 60b, through the terminal sleeve 138, across plate 134 and to the electrically conductive sealing plate 112 upon activation. As mentioned above, the outer insulative housing 114 of jaw member 110 effectively eliminates stray electrical currents and incidental burning of tissue across the intended electrical path.

As best shown in Fig. 4B, jaw member 110 includes a raceway 135 extending proximally from the flange 130 which includes terminal sleeve 138 at the proximal-most end thereof. The terminal sleeve 138 connects to the conductive tube 60b disposed within shaft 12b as described above. Raceway 135 serves two purposes: 1) to provide electrical continuity from the terminal sleeve 138, through the electrically conductive plate 134 and to the electrically conductive sealing surface 112; and 2) to provide a channel for guiding lead 210a to the distal connector 300 as described below.

Insulated outer housing 114 is dimensioned to securely engage the electrically conductive sealing surface 112. It is envisioned that this may be accomplished by stamping, by overmolding, by overmolding a stamped electrically conductive sealing plate and/or by overmolding a metal injection molded seal plate. All of these manufacturing techniques produce an electrode having an electrically conductive surface 112 which is substantially surrounded by an insulated outer housing 114.

It is envisioned that the jaw member may also include a second insulator (not shown) disposed between the electrically conductive sealing surface 112 and the outer insulative housing 114. The insulated outer housing 114 and the electrically conductive sealing surface 112 (and the other insulator if utilized) are preferably dimensioned to limit and/or reduce many of the known undesirable effects related to tissue sealing, e.g., flashover, thermal spread and stray current dissipation.

It is also envisioned that the electrically conductive sealing surface 112 may include a pinch trim (not shown) which facilitates secure engagement of the electrically conductive surface 112 to the insulated outer housing 114 and also simplifies the overall manufacturing process. It is also contemplated that the electrically conductive sealing surface 112 may include an outer peripheral edge which has a radius and the insulated outer housing 114 meets the electrically conductive sealing surface 112 along an adjoining edge which is generally tangential to the radius and/or meets along the radius. Preferably, at the interface, the electrically conductive surface 112 is raised relative to the insulated outer housing 114. These and other envisioned embodiments are discussed in concurrently-filed, co-pending, commonly assigned Application Serial No. 10/474,168 entitled "ELECTROSURGICAL INSTRUMENT WHICH REDUCES COLLATERAL DAMAGE TO ADJACENT TISSUE" by Johnson et al. published as US 2005 021025.

As best illustrated in the exploded view of Fig. 4B, the inner periphery of tube 60b is preferably dimensioned to house lead 210a therethrough such that a different electrically potential can be effectively transmitted to jaw member 120. More particularly and as mentioned above, cable 210 houses two leads 210a and 210b having different electrical potentials. The first lead 210a is disposed through tube 60b and conducts the first electrical potential to jaw member 120 as described in more detail below. The second lead 210b is electrically interfaced with tube 60b at a proximal connector 80 (Fig. 7) which includes a series of electrical crimps 85, 87 and 89 for securing lead 210b to tube 60b. As a result, tube 60b carries the second electrical potential therethrough for ultimate connection to jaw member 110 as described above.

Lead 210a preferably includes an insulative coating 213 which surrounds an inner core or electrical conductor 211 (e.g., wire) disposed therein to insulate the electrical conductor 211 from the tube 60b during activation. It is envisioned that the wire 211 may be made from a solid or multi-strand electrically conductive material, e.g., copper/aluminum, which is surrounded by an insulative, non-conductive coating 213 , e.g., plastic.

The wire 211 includes a terminal end 212 which is dimensioned to electrically interface with jaw member 120. Preferably, the terminal end 212 is "flat-formed" in a generally arcuate shape to encircle a corresponding boss 314 which extends upwardly from the distal connector 300 towards jaw member 120 as described below. It is envisioned that the distal connector 300 performs at least two functions: 1) to insulate jaw member 110 from jaw member 120; and 2) to provide a running electrical connection for lead 210a to jaw member 120.

More particularly, the distal connector 300 is generally shaped to match the overall profile of the electrically conductive face plates 134 and 144 of jaw members 110 and 120, respectively, such that, upon assembly, outer facing surfaces 302 and 304 of the distal connector 300 abut against the corresponding plates 134 and 144 of jaw member 110 and 120, respectively. It is envisioned that the outer facing surface 302 of the distal connector 300 acts as a runway surface which facilitates pivotable motion of jaw member 120 about pivot pin 151 relative to jaw member 110. Preferably, the distal connector 300 is made form an insulative substrate such as plastic or some other non-conductive material.

The distal connector includes a series of flanges 322 and 326 which extend towards jaw member 120 and a second series of flanges 324 and 328 which extend towards jaw member 110. It is envisioned that these flanges 322, 324, 326 and 328 insulate the other operative components of the forceps 10 and the patient from stray electrical currents emanating from the electrically conductive plates 134 and 144 during activation. Flanges 322 and 328 may also be dimensioned to limit / restrict the expansion of tissue 400 beyond the sealing surfaces 112 and 122 during activation. Flanges 326 and 324 are preferably dimensioned to insulate the forceps during all angles of operation, i.e., pivoting of the jaw members 110 and 120.

As mentioned above, the distal connector 300 includes a boss 314 which extends towards jaw member 120 which is dimensioned to secure the terminal end 212 of lead 210a. Preferably, the boss is designed to electrically insulate the terminal end of the lead from the pivot. The boss 314 preferably defines an aperture 316 therethrough for receiving the pivot pin 151 and to allow pivotable motion of jaw member 120 about the pivot 151 and the boss 314 relative to jaw member 110.

A continuous series of recesses 312, 318 and 319 are formed around and proximate boss 314 to seat the flat-formed terminal end 212, the wire 211 and the insulated portion of the lead 210a, respectively. This also secures lead 210a to the distal connector and limits movement of the same (210a). In some cases it may be preferable to include a dollop of silicone or other non-conductive material at the junction between the wire and the terminal end 212 as an added and/or alternative insulating safeguard. It is also envisioned that flange 326 may include a notch (not shown) disposed therethrough which facilitates assembly of the lead 210a atop the distal connector 300. As can be appreciated, this eliminates the step of forming the arcuately-shaped terminal end 212 after insertion through channel 318. As mentioned above, a dollop of silicone or the like may be added atop / within the notch for insulation purposes after the terminal end 212 is seated within the distal connector 300.

The proximal-most portion of distal connector 300 includes a finger 320 which is dimensioned to seat within a channel 137 formed within the raceway 135 such that the distal connector 300 moves in connection with jaw member 110 during pivoting. Channel 135 may be formed during a molding process, subsequently bored after the raceway 135 is formed or by any other known method of formation. The uppermost edge of boss 314 is preferably dimensioned to seat within a corresponding recess (not shown) formed within plate 144. Likewise and although not shown, it is envisioned that the opposite end of boss 314 extends towards plate 134 and seats within a recess 131 formed within plate 134. It is envisioned that recess 131 promotes engagement of the distal connector 300 with the jaw member 110.

The distal connector 300 also includes a spring washer or wave washer 155 which is preferably dimensioned to encircle the boss 314 atop terminal end 212. Upon assembly, the washer 155 is sandwiched / wedged between the terminal end 212 and the conductive plate 144 of jaw member 120. It is envisioned that the washer 155 enhances the connection between the terminal end and the plate 144. More particularly, the washer 155 is preferably shaped such that the washer 155 provides a self-cleaning, running electrical contact between the terminal end 212 and the jaw member 120. It is contemplated that the washer 155 "self-cleans" due to the frictional contact and relative movement of the washer 155 with respect to the terminal end 212 during pivoting of the jaw members 110 and 120. The self-cleaning action can be attributed to the washer 155 rubbing, scoring and/or digging against the terminal end 212 and/or the plate 144 during pivoting of the jaw members 110 and 120.

The outer housing of each of the jaw members 110 and 120 preferably includes an additional recess or circular groove 129 which receives a ring-like insulator 153b and 153a, respectively. Insulators 153a and 153b insulate the pivot pin 150 from the jaw members 110 and 120 when the forceps 10 is assembled. Preferably, the pivot pin 150 is peened to secure the jaw members 110 and 120 during assembly and may include outer rims 151 a and 151 b at least one of which is peened or formed after the jaw members 110 and 120 are assembled about the pivot pin 150 as best shown in Fig. 4B.

Upon activation, the first electrical potential is carried by lead 210a through tube 60b to the terminal end 212. The washer 155 of the distal connector 300 then conducts the first potential to face plate 144 which carries the first potential to sealing plate 122 disposed on the inner facing surface of jaw member 120. The second potential is carried by lead 210b which electrically interfaces with the tube 60b (by way of crimps 85, 87 and 89) to conduct the second potential to terminal sleeve 138 of jaw member 110. The terminal sleeve 138 electrically connects to sealing surface 112 across face plate 134.

Figure 8 shows the connection of the cable 210 within the cavity 45b of shaft 12b. As mentioned above a series of finger-like elements 77a and 77b and crimps 76a and 76b secure the cable 210 within shaft 12b. Preferably, cable 210 is secured at an angle alpha (α) relative to a longitudinal axis "A" disposed along shaft 12b. It is envisioned that angling the cable 210 in an inward direction, i.e., towards shaft 12a, facilitates handling of the forceps 10 and the cable 210 during surgery, i.e., the angled disposition of the cable 210 as it exits the forceps 10 tends to reduce cable tangling and/or cable interference during handling.

Preferably at least one of the jaw members 110 and 120 includes a skirt-like feature 126 and 136, respectively, which is dimensioned to prevent exposure of the terminal end 212 or wire 211 during all angles of operation, i.e., when the jaw members 110 and 120 are disposed in the first open position, the second closed position and/or during operative movement therebetween.

It is envisioned that by making the forceps 10 disposable, the forceps 10 is less likely to become damaged since it is only intended for a single use and, therefore, does not require cleaning or sterilization. As a result, the functionality and consistency of the vital sealing components, e.g., the conductive surfaces 112 and 122, the stop member(s) 150, and the insulative housings 124 and 114 will assure a uniform and quality seal.

From the foregoing and with reference to the various figure drawings, those skilled in the art will appreciate that certain modifications can also be made to the present disclosure without departing from the scope of the present disclosure. For example, it may be preferable to include a tang which facilitates manipulation of the forceps 10 during surgery.

Moreover, although the electrical connections are preferably incorporated with the bottom shaft 12b and the instrument is intended for right-handed use, it is contemplated the electrical connections may be incorporated with the other shaft 12a depending upon a particular purpose and/or to facilitate manipulation by a left-handed user.

It is also contemplate that a shrink tube may be employed over the proximal connector 80 and/or the other various solder or crimp connections 85, 87 and 89 associated with the proximal connector 80 interface with 1ead wire 210b. This provides additional insulating protection during assembly. It is also contemplated that the forceps 10 (and/or the electrosurgical generator used in connection with the forceps 10) may include a sensor or feedback mechanism (not shown) which automatically selects the appropriate amount of electrosurgical energy to effectively seal the particularly-sized tissue 400 grasped between the jaw members 110 and 120. The sensor or feedback mechanism may also measure the impedance across the tissue during sealing and provide an indicator (visual and/or audible) that an effective seal has been created between the jaw members 110 and 120.

## Claims

1. A bipolar electrosurgical instrument (10) for use in open surgery, comprising:
first and second shafts (12a, 12b) each having a jaw member (110, 120) extending from a distal end thereof and a handle (17a, 17b) disposed at a proximal end thereof for effecting movement of the jaw members relative to one another from a first position wherein the jaw members are disposed in spaced relation relative to one another to a second position wherein the jaw members cooperate to grasp tissue therebetween, each of the jaw members including an electrically conductive sealing surface (112, 122);
a source (210) of electrical energy having a first electrical potential (210a) connected to one of the jaw members and a second electrical potential (210b) connected to the other of the jaw members such that the jaw members are capable of selectively conducting energy through tissue (400) held therebetween to effect a seal;
the first and second electrical potentials being transmitted to the jaw members through the first shaft wherein the first electrical potential is transmitted by a lead (210a) having a terminal end (212); and
at least one non-conductive stop member (150) disposed on the electrically conductive sealing surface of at least one of the jaw members which controls the distance between the jaw members when tissue is held therebetween;
**characterized in that**:
the terminal end of the lead electrically interfaces with a spring washer (155), the spring washer acting as an electrical intermediary between the terminal end and the jaw member,
and the spring washer (155) is dimensioned to rotate relative the terminal end during movement of the jaw members from the first to second positions to provide a self-cleaning, enhanced electrical contact between the terminal end and the jaw member.

2. The bipolar electrosurgical instrument according to claim 1, wherein the second electrical potential is transmitted through the first shaft by a tube (60a) disposed within the first shaft which connects the other jaw member to the second electrical potential.

3. The bipolar electrosurgical instrument according to any one of claims 1 and 2, further comprising an insulator (300) disposed between the jaw members for electrically isolating the first and second potentials.

4. The bipolar electrosurgical instrument according to claim 3, wherein the insulator is a distal connector (300) being made from an insulative substrate.

5. The bipolar electrosurgical instrument according to claim 4, wherein the distal connector includes a first surface (302) having at least one recess defined therein which is dimensioned to receive at least a portion of the terminal end.

6. The bipolar electrosurgical instrument according to any one of the preceding claims, wherein the terminal end includes a flat-formed wire (212).

7. The bipolar electrosurgical instrument according to any one of the preceding claims, wherein the spring washer is dimensioned to enhance the electrical interface between the terminal end and the jaw member.

8. The bipolar electrosurgical instrument according to any one of the preceding claims, wherein at least one of the jaw members includes a skirt (126, 136) which is dimensioned to prevent exposure of the terminal end when the jaw members are disposed in the first position, the second position and during operative movement therebetween.

9. The bipolar electrosurgical instrument according to claim 4, or any one of claims 5 to 8 as dependent on claim 4, wherein the lead is fed to the distal connector through the tube and the lead includes an insulative coating (213) which surrounds a wire-like electrical conductor to insulate the wire-like conductor from the tube during activation.

## Patentansprüche

1. Bipolares elektrochirurgisches Instrument (10) zur Verwendung bei offenen chirurgischen Eingriffen, umfassend:
einen ersten und zweiten Schaft (12a, 12b), die jeweils ein sich aus deren distalem Ende erstreckendes Backenelement (110, 120) und einen an deren proximalem Ende davon angeordneten Griff (17a, 17b) aufweist, um eine Bewegung der Backenelemente relativ zueinander aus einer ersten Position; in der die Backenelemente im beabstandeten Verhältnis relativ zueinander angeordnet sind, in eine zweite Position, in der die Backenelemente zusammenwirken, um Gewebe dazwischen zu greifen, zu bewirken, wobei jedes der Backenelemente eine elektrisch leitende Abdichtoberfläche (112, 122) umfasst;
eine Quelle (210) elektrischer Energie mit einem ersten elektrischen Potenzial (210a), das mit einem der Backenelemente verbunden ist, und mit einem zweiten elektrischen Potenzial (210b), das mit dem anderen der Backenelemente verbunden ist, so dass die Backenelemente in der Lage sind, selektiv Energie durch das dazwischen gehaltene Gewebe (400) zu leiten, um eine Abdichtung zu bewirken;
wobei das erste und zweite elektrische Potenzial durch den ersten Schaft zu den Backenelementen übertragen werden, wobei das erste elektrische Potenzial von einer Leitung (210a) mit einem Anschlussende (212) übertragen wird; und
mindestens ein nicht leitendes Anschlagselement (150), das auf der elektrisch leitenden Abdichtoberfläche mindestens eines der Backenelemente angeordnet ist und das den Abstand zwischen den Backenelementen steuert, wenn Gewebe dazwischen gehalten wird;
**dadurch gekennzeichnet, dass**:
das Anschlussende der Leitung mit einer Federscheibe (155) elektrisch verbunden ist, wobei die Federscheibe als elektrisches Zwischenglied zwischen dem Anschlussende und dem Backenelement wirkt,
und die Federscheibe (155) so bemessen ist, dass sie sich während der Bewegung der Backenelemente aus der ersten in die zweite Position relativ zum Anschlussende dreht, um einen sich selbst reinigenden, verbesserten elektrischen Kontakt zwischen dem Anschlussende und dem Backenelement bereitzustellen.

2. Bipolares elektrochirurgisches Instrument nach Anspruch 1, wobei das zweite elektrische Potenzial mit Hilfe eines Rohrs (60a), das im ersten Schaft angeordnet ist und das andere Backenelement mit dem zweiten elektrischen Potenzial verbindet, durch den ersten Schaft übertragen wird.

3. Bipolares elektrochirurgisches Instrument nach einem der Ansprüche 1 und 2, weiter einen Isolator (300) umfassend, der zwischen den Backenelementen angeordnet ist, um das erste und zweite Potenzial elektrisch zu isolieren.

4. Bipolares elektrochirurgisches Instrument nach Anspruch 3, wobei der Isolator ein distaler Verbinder (300) ist, der aus einem isolierenden Substrat hergestellt ist.

5. Bipolares elektrochirurgisches Instrument nach Anspruch 4, wobei der distale Verbinder einer erste Oberfläche (302) mit mindestens einer darin definierten Vertiefung umfasst, die so bemessen ist, dass sie mindestens einen Teil des Anschlussendes aufnimmt.

6. Bipolares elektrochirurgisches Instrument nach einem der vorangehenden Ansprüche, wobei das Anschlussende einen flach geformten Draht (212) umfasst.

7. Bipolares elektrochirurgisches Instrument nach einem der vorangehenden Ansprüche, wobei die Federscheibe so bemessen ist, dass sie die elektrischen Berührungsflächen zwischen dem Anschlussende und dem Backenelement erhöhen.

8. Bipolares elektrochirurgisches Instrument nach einem der vorangehenden Ansprüche, wobei mindestens eines der Backenelemente eine Randleiste (126, 136) umfasst, die so bemessen ist, dass sie eine Exposition des Anschlussendes verhindert, wenn die Backenelemente in der ersten Position, der zweiten Position sowie während der Arbeitsbewegung dazwischen angeordnet sind.

9. Bipolares elektrochirurgisches Instrument nach Anspruch 4 oder einem der Ansprüche 5-8, wenn abhängig von Anspruch 4, wobei die Leitung durch das Rohr zum distalen Verbinder geführt wird und die Leitung eine isolierende Ummantelung (213) umfasst, die einen drahtähnlichen elektrischen Leiter umgibt, um so den drahtähnlichen Leiter während der Aktivierung vom Rohr zu isolieren.

## Revendications

1. Instrument électrochirurgical bipolaire (10) pour utilisation en chirurgie ouverte, comprenant:
des première et seconde branches (12a, 12b), chacune ayant un élément de mâchoire (110, 120) s'étendant à partir d'une extrémité distale de celle-ci, et une poignée (17a, 17b) disposée à une extrémité proximale de celle-ci pour effectuer un mouvement des éléments de mâchoire l'un relativement à l'autre à partir d'une première position où les éléments de mâchoire se trouvent en une relation espacée l'un de l'autre à une seconde position où les éléments de mâchoire coopèrent pour saisir le tissu entre eux, chacun des éléments de mâchoire incluant une surface de scellement électriquement conductrice (112, 122);
une source (210) d'énergie électrique ayant un premier potentiel électrique (210a) relié à l'un des éléments de mâchoire et un second potentiel électrique (210b) relié à l'autre des éléments de mâchoire de sorte que les éléments de mâchoire sont aptes à conduire sélectivement l'énergie à travers le tissu (400) tenu entre eux pour effectuer une suture;
les premier et second potentiels électriques étant transmis aux éléments de mâchoire par la première branche, où le premier potentiel électrique est transmis par un conducteur (210a) ayant une extrémité terminale (212); et
au moins un élément d'arrêt non-conducteur (150) disposé sur la surface de scellement électriquement conductrice d'au moins l'un des éléments de mâchoire qui commande la distance entre les éléments de mâchoire lorsque le tissu est tenu entre eux;
**caractérisé en ce que**:
l'extrémité terminale du conducteur est en interface électrique avec une rondelle élastique (155), la rondelle élastique agissant comme intermédiaire électrique entre l'extrémité terminale et l'élément de mâchoire,
et la rondelle élastique (155) est dimensionnée pour tourner relativement à l'extrémité terminale pendant le mouvement des éléments de mâchoire de la première à la seconde position pour réaliser un plus grand contact électrique auto-nettoyant entre l'extrémité terminale et l'élément de mâchoire.

2. Instrument électrochirurgical bipolaire selon la revendication 1, où le deuxième potentiel électrique est transmis à travers la première branche par un tube (60a) disposé dans la première branche qui relie l'autre élément de mâchoire au second potentiel électrique.

3. Instrument électrochirurgical bipolaire selon l'une des revendications 1 et 2, comprenant en outre un isolateur (300) disposé entre les éléments de mâchoire pour isoler électriquement les premier et second potentiels.

4. Instrument électrochirurgical bipolaire selon la revendication 3, où l'isolateur est un connecteur distal (300) réalisé par un substrat isolant.

5. Instrument électrochirurgical bipolaire selon la revendication 4, où le connecteur distal comprend une première surface (302) ayant au moins un évidement défini dans celle-ci qui est dimensionné pour recevoir au moins une portion de l'extrémité terminale.

6. Instrument électrochirurgical bipolaire selon l'une des revendications précédentes, où l'extrémité terminale comprend un fil de forme plate (212).

7. Instrument électrochirurgical bipolaire selon l'une des revendications précédentes, où la rondelle élastique est dimensionnée pour augmenter l'interface électrique entre l'extrémité terminale et l'élément de mâchoire.

8. Instrument électrochirurgical bipolaire selon l'une des revendications précédentes, où au moins l'un des éléments de mâchoire comprend une collerette (126, 136) qui est dimensionnée pour empêcher l'exposition de l'extrémité terminale lorsque les éléments de mâchoire se trouvent dans la première position, la seconde position et pendant le mouvement fonctionnel entre celles-ci.

9. Instrument électrochirurgical bipolaire selon la revendication 4, où l'une des revendications 5 à 8 dépendant de la revendication 4, où le conducteur est amené au connecteur distal à travers le tube, et le conducteur comprend un revêtement isolant (213) qui entoure un conducteur électrique en forme de fil pour isoler le conducteur en forme de fil du tube pendant l'activation.
